# EUROPEAN PATENT APPLICATION

(11) **EP 2 607 493 A1**
(43) Date of publication of application: **26.06.2013**
(21) Application number: 11195537.3
(22) Date of filing: 23.12.2011
(51) Int. Cl.: C12Q 1/68, G01N 33/576

(54) **Diagnosis of steatohepatitis**

(71) Applicant: IMG Institut für medizinische Genomforschung Planungsgesellschaft M.B.H., 1010 Vienna (AT)
(72) Inventor: Starmann, Julia, 69118 Heidelberg (DE); Sültmann, Holger, 67117 Limburgerhof (DE); Fälth, Maria, 26393 Höganäs (SE); Zatloukal, Kurt, 8047 Graz (AT); Trauner, Michael, 8010 Graz (AT); Lackner, Karoline, 8062 Kumberg (AT)
(74) Representative: Sonn & Partner Patentanwälte

(57) **Abstract**

The invention discloses the use of keratin 23 (KRT23) as a marker for distinguishing between steatosis and steatohepatitis.

## Description

The invention relates to biomarkers for distinguishing steatohepatitis from steatosis.

Fatty liver diseases comprise a spectrum of severity ranging from simple steatosis over steatohepatitis to cirrhosis and hepatocellular cancer (HCC). There are two major etiologies for fatty liver disease, namely alcohol and metabolic disorders such as obesity and type 2 diabetes mellitus (T2DM). Due to its high prevalence and potential for severe hepatic outcomes such as liver cirrhosis and HCC in a substantial fraction of affected individuals, fatty liver disease has become a major issue for the society and health care. Up to 30% of the general population is affected by non-alcoholic fatty liver disease (NAFLD), reaching up to 70% among diabetic patients. The prevalence of steatosis and steatohepatitis in obese patients undergoing bariatric surgery is as high as 76% and 37%, respectively. Steatohepatitis develops in about 20% of alcoholics and up to 50% of T2DM who are also obese (BMI > 30). This places fatty liver disease as the most common liver disease of the 21^{st} century accounting for the majority of liver cirrhosis and HCC in Western countries. Its prevalence is expected to further rise in light of the ongoing epidemic of diabetes and obesity.

While simple steatosis has a relatively benign course and is principally reversible, steatohepatitis carries a poor prognosis and can lead to severe liver damage with progression to cirrhosis and HCC. Conventional non-invasive markers such as serum transaminases correlate poorly with the risk of development as well as progression of liver disease, and currently available routine liver tests may even be unremarkable in a significant proportion of patients with steatohepatitis. Therefore, in current standard clinical practice, non-invasive serum and imaging markers do not allow the distinction of relatively benign fatty liver from progressive steatohepatitis. This situation results in underdiagnosis and undertreatment of these disorders. The development of efficient diagnostic, prognostic and therapeutic strategies has been substantially hampered by the fact that the understanding of the molecular pathogenesis of steatohepatitis is still incomplete. Several studies showed that the different forms of steatohepatitis (alcoholic - ASH, non-alcoholic - NASH) cannot be morphologically distinguished, which suggests a common pathogenetic mechanism despite different etiologies of the disease. According to current concepts, deregulation of energy metabolism may lead to simple steatosis, which needs to be accompanied by a second inflammatory insult to lead to steatohepatitis. A major unsolved problem is the marked difference in the individual risk to develop steatohepatitis and to progress to cirrhosis (e.g., only 20% of heavy drinkers or 50% of obese type II diabetic patients develop steatohepatitis; Hispanics and Caucasians are more susceptible than Afro-Americans). However, the factors responsible for disease progression across the spectrum of fatty liver disease are poorly understood. Why some patients are protected against developing steatohepatitis or simple steatosis, while others are not, is still unclear. It is currently even debated whether steatosis and steatohepatitis represent two consecutive disease stages; alternatively, individuals may *a priori* be predetermined to develop either a rather benign steatosis or prognostically dismal steatohepatitis.

Diagnosis of steatohepatitis is difficult and currently only possible by liver biopsy (for example, NASH refers to findings on liver biopsy in patients with steatohepatitis in the absence of significant alcohol consumption). Therefore, liver biopsy remains the only means of assessing the presence and extent of specific necroinflammatory changes and fibrosis in steatohepatitis. However, firm recommendations of when to perform a liver biopsy in the routine clinical setting have not yet been developed. The use of surrogate markers, such as aminotransferases and fibrosis markers, are not adequate for monitoring diseases as is necessary for clinical studies. In US 2009/0304704 A1 various expression markers have been suggested for diagnosing a NASH disease state in a patient comprising determining a level of expression of a panel of more than 30 genes associated with the onset or progression of NASH in a patient sample and comparing the level of expression (an expression "profile") with a predetermined value for NASH-associated gene expression in this panel of genes and correlating the level of expression with a NASH disease state. Such profile determination is complicated and difficult to establish in routine diagnosis and testing large numbers of samples. There is still a need for further, single markers which can be used instead of profile determination for diagnosing steatohepatitis, especially for distinguishing between steatosis and steatohepatitis.

Moreover, it is also desirable, if such single markers are not only useful in biopsy material but also in non-invasive specimen, such as body fluids which can be easily taken from patients, such as blood or urine, or smears from various mucosae.

Accordingly, neither suitable tissue sample testing nor a suitable serum diagnostic tests which can easily be transformed to a routine method are available. This represents a huge unmet medical need (US 2009/0304704 A1).

It is therefore an object of the present invention to provide a suitable biomarker which allows a reliable, preferably also non-invasive, diagnosis of steatohepatitis, especially differentiation between steatosis and steatohepatitis. The present invention is specifically aimed to provide means and methods for differentiation between the clinically "benign" simple steatosis and steatohepatitis that might progress to liver cirrhosis and liver cancer.

Therefore, the present invention provides the use of keratin 23 (KRT23) as a marker for distinguishing between steatosis and steatohepatitis.

It was shown with the present invention that KRT23 is a highly reliable molecular marker for distinguishing steatosis from steatohepatitis. Moreover, KRT23 can also be used for monitoring the development from steatohepatitis to HCC. KRT23 can also be used together with other markers for steatohepatitis and/or HCC for further optimising diagnosis of such liver diseases.

KRT23 is specifically suitable for diagnosing NASH, since KRT23 levels are significantly increased in NASH patients compared to healthy individuals or individuals having steatosis.

The method according to the present invention is advantageous compared to current diagnosis (grading, staging) of steatohepatitis which relies on liver biopsy as a diagnostic gold standard for differentiation between "simple" steatosis and (N)ASH. Routine biochemical serum tests (liver function tests) currently available underestimate the occurrence and severity of steatohepatitis. Although the degree of liver fibrosis may be a crude predictor for the development of liver cirrhosis, more sophisticated individual risk markers/profiles for the development of cirrhosis and hepatocellular cancer are required. Moreover, the relative contribution of cardiovascular versus liver-related morbidity/risk may vary significantly among individual NAFLD/NASH patients. Given the high prevalence of steatosis and steatohepatitis in the general population, there is an urgent need for diagnostic tests and prognostic biomarkers (especially for such markers which can be diagnosed in a non-invasive manner, i.e. without the need for liver tissue samples) which predict the individual disease course and need for and response to therapy

A specific aspect of the present invention relates to a method for diagnosing steatohepatitis in a sample of a human body fluid or a human tissue sample comprising the determination of the amount of KRT23 or KRT23 m-RNA in this sample and diagnosing steatohepatitis, if the amount of KRT23 or KRT23 m-RNA is increased compared to a sample of this human body fluid or tissue sample from a person with no steatohepatitis.

Preferably, the body fluid is blood or a blood derived sample, preferably a serum or a plasma sample. This makes the present method a suitable non-invasive method which can be used even without previous taking of biopsies, especially liver biopsies which may constitute a certain risk factor. Measuring KRT23 in blood or a sample derived from a blood sample is easily adaptable to larger numbers of samples and may therefore be established for routine testing (e.g. as an antibody test or a PCR test (or as a routing MALDI-test)).

On the other hand, the present method can also routinely be applied for tissue samples, especially liver tissue samples (e.g. taken from liver surgery) or liver biopsy samples (e.g. taken by usual liver biopsy needles).

Since KRT23 antibodies are available, antibody based test methods according to the present invention can easily be established. It is therefore preferred to detect the amount of KRT23 by a KRT23 antibody.

Alternatively, detecting the amount of KRT23 m-RNA by a KRT23 m-RNA binding nucleic acid is another preferred embodiment of the present invention.

The present invention refers to KRT23 for use in a method for diagnosing steatohepatitis.

KRT23 is a gene/protein which is widely known and investigated; detection and quantification of expressed KRT23-m-RNA or KRT23-protein is therefore a standard technique well available to a person skilled in the art. Preferably, the expression or amount of KRT23 in the body fluid sample or in the tissue specimen, especially in a liver biopsy sample or a liver tissue sample identified, is determined by immunohistochemical (IHC) methods, by immunofluorescence (IF) methods, by RNA in-situ hybridisation, by reverse transcriptase polymerase chain reaction (RTPCR), especially quantitative real time RT-PCR (qRT-PCR), or by a combination of these methods. Such samples could preferably be taken by manual or microscopical microdissection. Other methods for determining the level of (expression of) KRT23 include MALDI-MS (including surface enhanced laser desorption/ionization mass spectrometry (SELDI-MS), especially surface-enhanced affinity capture (SEAC), surface-enhanced need desorption (SEND) or surface-enhanced photo label attachment and release (SEPAR) for the determination of proteins in samples for diagnosing and staging of steatohepatitis), antibody testing (including immunoprecipitation, Western blotting, Enzyme-linked immuno sorbent assay (ELISA), Enzyme-linked immuno sorbent assay (RIA), dissociation-enhanced lanthanide fluoro immuno assay (DELFIA), scintillation proximity assay (SPA) for diagnosing, staging or monitoring steatohepatitis samples under investigation of specific marker (KRT23) proteins), and quantitative nucleic acid testing, especially PCR, LCR and RT-PCR of samples for marker (KRT23) mRNA detection and quantification.

Preferred detection agents for KRT23 are antibodies being specific for KRT23. Such antibodies are well available in the art; commercial anti-KRT23-antibodies can be purchased also in high purity levels to establish suitable diagnostic kits. The antibodies to be used according to the present invention can be monoclonal antibodies as well as polyclonal antibodies, i.e. antibodies contained in a polyclonal antiserum, as well as any antibody fragment wherein the binding specificity for KRT23 is present. Suitable fragments of antibodies include F(ab')2, Fab and Fv fragments. Suitable derivatives of antibodies include scFvs, chimeric and humanized antibodies. Also diagnostic monoclonal antibodies from mice can be applied according to the present invention. These are easy and cost effective to be produced in cell cultures and can easily be standardised for routine testing.

Usually, the amount of KRT23 protein or the amount of KRT23 mRNA in the sample is compared with a reference value for this amount with a known status concerning steatohepatitis and/or steatosis, i.e. a value which is known to be a healthy value (i.e. a value not affected by steatohepatitis) or which is known to be a diseased status with respect to steatohepatitis/steatosis, preferably of a given/defined stage of steatohepatitis.

Such reference, standard or control samples are all useable in principle for comparison with the sample of unknown status concerning steatohepatitis and/or steatosis diagnosed according to the present invention. Such reference, standard or control samples can be taken e.g. from a human subject with negative diagnosis concerning steatohepatitis and/or steatosis or undetectable ongoing steatohepatitis development. If such a control sample, standard sample or reference sample is said to be comparable to the sample that is taken from a human subject being suspected to be afflicted by ongoing steatohepatitis development according to the present invention, this means that both samples, except for their expression profile have been derived and treated equally. Thus, the sample in both cases may e.g. be a tissue or blood derived sample which has been further treated in a way to allow for detection of the diagnostic marker molecules as mentioned above.

Although liver tissue samples from the patient are preferred samples, the present marker is also useable in other suitable samples of tissue or body fluids. For example, the sample may be a blood sample, or a sample derived from blood, such as e.g. a serum sample or a plasma sample or a fraction of a blood, serum or plasma sample. Samples which do not need liver biopsies or liver puncture performed on the patient for providing the samples are specifically preferred for early staging and diagnosis of steatohepatitis.

Blood plasma is the yellow liquid component of blood, in which the blood cells in whole blood would normally be suspended. It makes up about 55% of the total blood volume. It is mostly water (90% by volume) and contains dissolved proteins, glucose, clotting factors, mineral ions, hormones and carbon dioxide. Blood plasma is prepared by spinning a tube of fresh blood in a centrifuge until the blood cells fall to the bottom of the tube. The blood plasma is then poured or drawn off. Blood plasma has a density of approximately 1.025 kg/l. Blood serum is blood plasma without fibrinogen or the other clotting factors (i.e., whole blood minus both the cells and the clotting factors).

The diagnostic methods according to the present invention may also be carried out in saliva, bladder washing, semen or urine samples, especially urine samples.

Although the diagnostic fine tuning for established clinical practice will work with nomograms for defining the differences in marker expression according to the present invention, it is also preferred to establish the diagnostic system with the comparison of the sample to be analysed according to the present invention and a sample of the same source of a known status. It is therefore preferred to use the amounts present of KRT23 (or the amount of respective KRT23 mRNA present) in a healthy (liver) tissue sample and compare these to the (liver) tissue sample under investigation. If the amount of KRT23 (or the mRNA) is significantly increased, diagnosis of steatohepatitis is indicated. The same is preferred for e.g. blood, plasma, serum, urine, semen or saliva samples: also here suitable comparison values are derived from samples blood, plasma, serum, urine, semen or saliva, respectively, from a patient with has a healthy status, preferable samples taken (earlier) from the same patient. On the other hand, the comparison may also be taken to establish known amounts of KRT23 (or the mRNA) in the sample taken. As usual in human medical diagnosis, absolute limiting value can be defined for each of the samples to determine difference between healthy (steatosis) and steatohepatitis and between different stages steatohepatitis or even HCC. These absolute values have to be defined and carefully confirmed depending on the very method applied for determining KRT23. For the examples according to the present invention, a reliable test system has been established.

For immunohistochemical analysis, e.g. paraffin sections of liver tissue of one case each of chronic hepatitis C, NAFLD associated steatosis (NAFL) and steatohepatitis can be dewaxed and rehydrated following standard procedures. For antigen retrieval the sections can be microwaved (e.g. in Target Retrieval Solution, pH 9.0 (Dako REAL TM S2367; Dako, Glostrup, Denmark), for 40 min at 160 W followed by cooling down for 20 min at RT). The sections may then be then washed in water and PBS. Blocking can be carried out (e.g. with Dako REAL TM Blocking Solution for 10 min prior to incubation with antibodies against KRT23 (e.g. diluted 1:500 in Dako REAL TM Antibody Diluent for 60 min at RT)). Binding of the antibodies can be detected by usual methods, e.g. with colourigenic reagents, such as the Dako REAL TM EnVision TM Detection System Peroxidase/DAB+. KRT23 protein expression in the cytoplasm of hepatocytes can be assessed semiquantitatively. The intensity of immunostaining can be classified as mild to moderate or marked, and the amount of positive hepatocytes can e.g. be estimated by application of numerical scores, for example defined as: Score 0 - KRT23 immunostaining in hepatocytes not detected; Score 1 - KRT23 positive hepatocytes comprise less than 10% of liver parenchyma; Score 2 - KRT23 positive hepatocytes comprise between 10-30% of liver parenchyma; Score 3 - KRT23 positive hepatocytes comprise more than 30% of liver parenchyma.

The level of expression or the amount of protein present of KRT23 according to the present invention is measured and is compared with the level of expression of KRT23 from other cells or samples. The comparison may be effected in an actual experiment; or intellectually (by comparison with known reference values) ; or virtually (e.g. automatically in silico). When the expression level (also referred to as expression pattern or expression signature (expression profile)) is measurably different, there is according to the invention a meaningful (i.e. statistically significant) difference in the level of expression. Preferably the difference in protein amount or in expressed mRNA for KRT23 is at least 5 %, 10% or 20%, more preferred at least 50% or may even be as high as 75% or 100%. More preferred this difference in the level of expression or protein amount is at least 200%, i.e. two fold, at least 500%, i.e. five-fold, or at least 1000%, i.e. 10 fold. The expression level for KRT23 according to the present invention expressed higher in a disease sample than in a healthy, normal (steatosis) sample is at least 5 %, 10% or 20%, more preferred at least 50% or may even be 75% or 100%, i.e. 2-fold higher, preferably at least 10-fold, higher in the disease (steatohepatitis) sample. Whether a KRT23 level is increased in a given detection method can preferably be established by analysis of a multitude of steatohepatitis samples with the given detection method. This can then form a suitable level from which the "increased" status can be determined; e.g. by the above % difference or -fold change. An increased expression of KRT23 is indicative for steatohepatitis. Since with usual methods, no KRT expression is detectable in healthy (steatosis) samples, any detection with normal test systems is already an "increased" level within the meaning of the present application. When more sophisticated and more sensitive tests are applied which identify (minimal) KRT23 expression in "healthy" status samples (i.e. below the noise ratio in current "normal" test systems), an "increased" expression of KRT23 may be established for a given test set-up as outlined above.

Within the course of the present invention, it was shown that the methionine-metabolism is compromised in steatohepatitis. This pathway is linked to gene regulation through modification of methyltransferase-activity via changes in S-adenosyl-methionine levels, leading to a modification of the activity of steatohepatitis-linked genes via alterations in histone- and DNA-methylation. As increased cell death rates are a key feature in advanced steatohepatitis, leading to the release of genomic DNA, DNA with such characteristic methylation signatures may be detectable also in the circulation.

These two observations now provide the rationale that the overexpression of KRT23 mRNA is due to altered DNA methylation and that altered methylation of the KRT23 promoter can be detected as free circulating DNA in serum or plasma.

A diagnostic assay for altered KRT23 methylation can be based on the detection of free circulating DNA the KRT23 promoter methylation in surgically resected or explated human livers (comparison of normal liver with simple steatosis and steatohepatitis). Based on the type of methylation patterns detected an assay for free circulating DNA can be established (e.g. as generally disclosed in WO 2008/103761 A2). This assay can be for example a methylation-specific PCR or sequencing of specifically enriched free circulating DNA that has been treated with bisulfite.

For further validation, gene expression analysis of KRT23 in human serum samples is performed. RNA in serum is present in low concentration and it is highly degraded. Therefore, effective methods are mandatory to improve both, the quality and the amount of the RNA. To enhance the quantification of KRT23 in serum, the extracted RNA fragments can be amplified. Then qRT-PCR and sequence-based analysis can be applied to quantify mRNA and miRNA abundance in serum samples and correlate these with the expression of the respective molecules in tissues. Additionally, it can be screened for circulating miRNA in the same serum samples to identify candidate miRNAs that regulate KRT23.

Accordingly, a diagnostic assay in serum using KRT23 expression as a signature for steatohepatitis is provided according to the present invention.

Another important aspect of the present invention is a kit for detecting the amount of KRT23 in a sample, comprising detection agents for KRT23, especially for use in diagnosing steatohepatitis and/or for distinguishing between steatosis and steatohepatitis. The present invention therefore also relates to the use of a kit for detecting the expression level of KRT23 in a sample, comprising detection agents for KRT23 mRNA for diagnosing a tissue sample or a sample of a body fluid for steatohepatitis. The present invention also relates to the use of a kit for detecting the amount of KRT23 in a sample, comprising detection agents for KRT23 for diagnosing a tissue sample or a sample of a body fluid for steatohepatitis.

The diagnostic kit for diagnosing steatohepatitis in a tissue sample or a sample of a body fluid according to the present invention comprises:
- a KRT23 m-RNA binding agent, preferably a KRT23 m-RNA binding nucleic acid and/or
- a KRT23 binding agent, preferably a KRT23 antibody.

In general, the kit according to the present invention includes binding agents that specifically bind to KRT23 protein. These binding agents are preferably linkable or already operably linked to a detectable label. An increased binding of the binding agent specifically bound to KRT23 protein in the sample of the patient compared to the binding agent bound to KRT23 protein in a sample of known steatohepatitis status, e.g. a sample from a healthy individual, indicates the steatohepatitis status of the patient. Such standard samples or other KRT23 standards are preferably further constituents of the present kit. In this embodiment of the invention, a medical imaging device or system detects the binding agent specifically bound to KRT23. Preferably, the binding agent is immobilised on a solid surface in order to allow simple confirmation of the binding event. Examples for such solid surfaces include microtiter plates, microarrays, ELISA plates, etc.. Exemplary binding agents include natural ligands, synthetic small molecules, chemicals, nucleic acids, peptides, proteins, antibodies and fragments thereof. As already stated above, antibodies against KRT23 and fragments thereof specifically binding to KRT23 are preferred binding agents according to the present invention.

Exemplary detectable labels include fluorophores, chemical dyes, radioactive compounds, chemiluminescent compounds, magnetic compounds, paramagnetic compounds, enzymes that yield a coloured product, enzymes that yield a chemiluminescent product and enzymes that yield a magnetic product. These labels may be linked to a secondary binding agent, e.g. an antibody specifically recognising the KRT23 binding agent. Various methods for detection of KRT23 are disclosed in the art or - taken from already established markers for liver tissue samples - easily adaptable to KRT23.

According to a preferred embodiment, the KRT23 binding agents are detectable by a different label than other markers which are tested, preferably detectable in one and the same assay, e.g. by different fluorophores, different dyes, different colour developing agent systems, etc..

According to a preferred embodiment for determining the amount of KRT23 in a liver tissue biopsy sample, KRT23 and each additional marker can be detected in consecutive tissue slices (preferably about 4-40 µm thickness). Alternatively, a multiple staining can be performed via immunofluorescence.

According to an alternative embodiment of the present invention, a kit for detecting the expression level of KRT23 in a sample is provided, comprising detection agents for KRT23 mRNA.

The kit can comprise suitable primer pairs specific for KRT23 mRNA and/or reagents enabling a quantitative detection of KRT23 mRNA in a given sample. The kit may also contain instructions for determining steatohepatitis diagnosis and prognosis based on the detection of the particular marker combination according to the present invention. The present kits are specifically useful for diagnosing a tissue sample or a sample of a body fluid for steatohepatitis.

The kit may alternatively or in combination also comprise specific oligonucleotides hybridising to KRT23 mRNA. Such specific oligonucleotides may act as probes for the KRT23 mRNA in the sample and can be used to quantitate the specific mRNA in the sample. Preferably, the oligonucleotide probes can be immobilised, e.g. on a suitable microarray. Also established microarray systems, as e.g. the Affymetrix chip, can be used for the diagnosis according to the present system.

Amplification assays, such as PCR, especially qRT-PCR, may be adapted for real time detection of multiple amplification products (i.e., multiplex real time amplification assays). Suitable primer pairs can be made based on the known sequences of the KRT23 mRNA available e.g. from NCBI, HGNC, etc.).

Such a kit may further include additional components such as additional oligonucleotides or primers and/or one or more of the following: buffers, reagents to be used in the assay (e.g., wash reagents, polymerases or internal control nucleic acid or cells or else) and reagents capable of detecting the presence of bound nucleic acid probe or primers. Examples of detection reagents include, but are not limited to radiolabelled probes, enzymatic labelled probes (horse radish peroxidase, alkaline phosphatase), and affinity labelled probes (biotin, avidin, or steptavidin). Of course the separation or assembly of reagents in same or different container means is dictated by the types of extraction, amplification or hybridization methods, and detection methods used as well as other parameters including stability, need for preservation etc. It will be understood that different permutations of containers and reagents of the above and foregoing are also covered by the present invention. The kit may also include instructions regarding each particular possible diagnosis, prognosis, theranosis or use, by correlating a combined KRT23 mRNA level over the KRT23 mRNA level with a particular diagnosis, prognosis, theranosis or use, as well as information on the experimental protocol to be used.

The present invention is further illustrated by the following examples and the drawing figures, yet without being limited thereto.

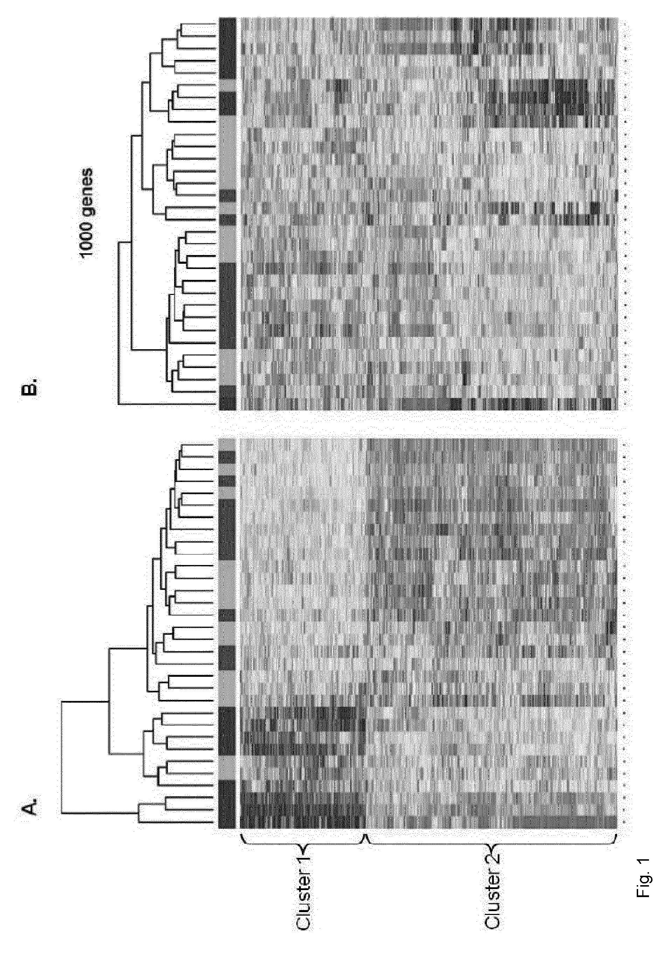

Figure 1 shows supervised (A) and unsupervised (B) clustering of differentially expressed genes in the three groups of liver samples. A. Common differentially expressed genes were used for supervised clustering. B. Unsupervised clustering was performed with the 1000 most variable genes in the three groups of liver samples (steatohepatitis, red; steatosis, orange; controls, green).

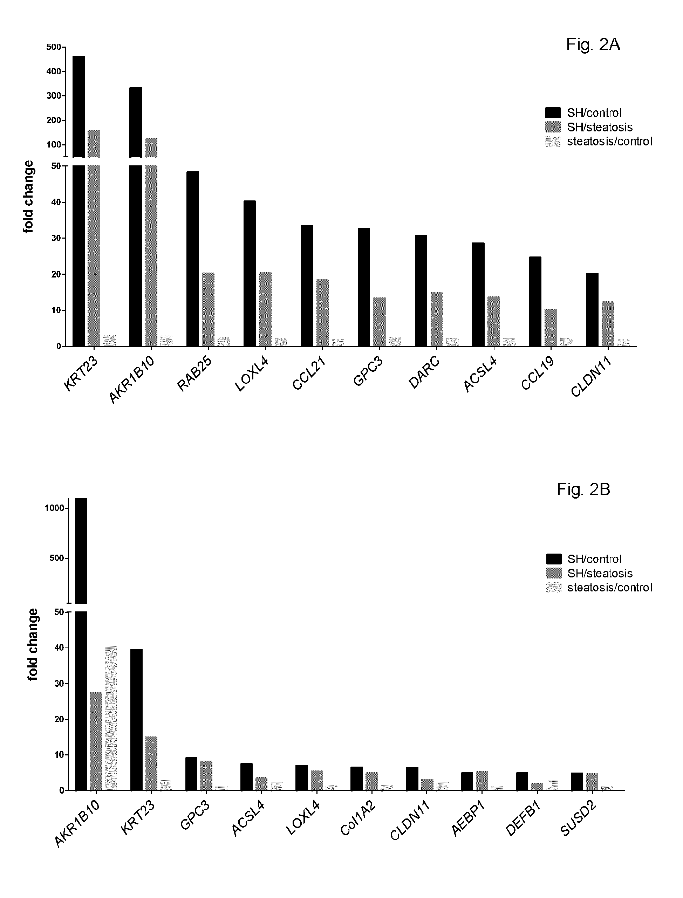

Figure 2 shows fold changes of selected genes validated by qRT-PCR. The expression of selected target genes were determined in surgically collected samples (A) and in liver samples obtained by biopsy (B) . The fold change was calculated by comparing the three groups of liver samples against each other.

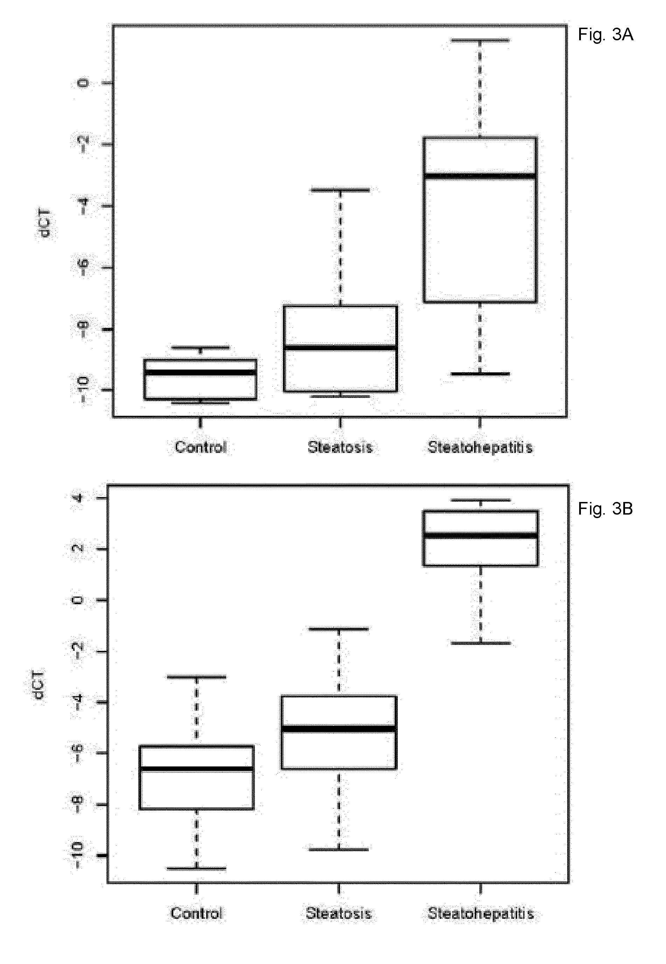

Figure 3 shows validation of KRT23 mRNA expression by qRTPCR in biopsy (A) and biobank (B) samples. HPRT1 was chosen to normalize the gene expression in the analyzed samples. Mean normalized expression levels are given in log2.

### Examples:

In the present examples, a microarray-based gene expression profiling analysis of steatosis and steatohepatitis was performed and compared them with normal human liver samples. It was focused on analysing transcriptional changes of genes relevant in steatohepatitis but not in steatosis and normal liver to identify potential signatures as basis for further development of biomarkers in the discrimination of steatohepatitis as a prognostically more relevant disease entity. Overall, 46 target genes were validated in human liver samples from two cohorts. A hitherto unknown molecular signature for premalignant changes in steatohepatitis but not steatosis was demonstrated. Several genes were highly significantly expressed in steatohepatitis compared to steatosis and normal liver. The most striking example was KRT23 according to the present invention. Besides the identification and verification of KRT23 as a suitable marker for steatohepatitis, the gene signature reported in the present examples serves as a valuable tool to distinguish between steatosis and steatohepatitis for future development of diagnostic and prognostic biomarkers.

### Materials and Methods

### Patients, tissue samples and experimental procedures Patient tissue samples

The detailed clinical, biochemical and histological data of the studied patients are given in table 1. The study was approved by the ethical review committee at the University of Graz (EK number: 20-119 ex 08/09). The diagnoses of all samples used were histologically validated by a board certified pathologist (C.L.) prior to molecular analysis. For histological analysis, hematoxylin and eosin (H&E) and chromotrope aniline blue (CAB) stained sections were used. A histological diagnosis of steatosis was made if more than 5% of the parenchymal area was occupied by steatosis using routine H&E stain, whereas the diagnosis of steatohepatitis (SH) was based on the presence hepatocellular ballooning in combination with variable degrees of steatosis and/or inflammation (Neuschwander-Tetri et al., Hepatology 37 (2003): 1202-1219). Stage of liver fibrosis was assessed according to the NASH Clinical Research Network Scoring System for NAFLD (Kleiner et al., Hepatology 41 (2005): 1313-1321).

Eighty-seven samples from two independent cohorts of human liver samples with alcoholic and non-alcoholic fatty liver disease were used for this study (table 1). The first cohort consisted of 58 tissue surgical samples (controls n=18; steatosis n=30; steatohepatitis n=10, 8/2 cirrhotic/non-cirrhotic) obtained from patients undergoing liver surgery for HCC (n=7), other malignancy/metastatic disease (n=33, 20/33 colon cancer), benign tumours of the liver (n=7) and organ dedicated to transplantation (explants, n=11). Virtually normal control samples in this cohort were taken from patients undergoing surgical resection of liver metastasis, but the surrounding liver tissue used for the molecular analyses was normal, non-tumorous liver tissue without detectable pathological changes. These samples (hereinafter termed 'Biobank cohort') were provided by the Biobank at the Medical University of Graz. The second cohort ('Biopsy cohort') encompassed 29 percutaneous liver biopsy samples obtained from patients at the Division of Gastroenterology and Hepatology, Department of Internal Medicine, Medical University of Graz (steatosis n=13; steatohepatitis n=11 (5/6 cirrhotic/non-cirrhotic), chronic hepatitis C (CHC) n=5 as disease controls with absence of steatosis). In this cohort CHC samples were used as controls since patients with normal liver tissue do not undergo percutaneous liver biopsy. Informed consent was obtained from all patients prior to use of an aliquot of the liver biopsy tissue for molecular analysis.

### RNA extraction and quality control

Total RNA was isolated from the samples using TRI Reagent® (Molecular Research Center, Cincinnati, OH, USA) according to the manufacturer's protocols. The RNA quality was analyzed using microcapillary electrophoresis (Agilent 2100 Bioanalyzer, Agilent Technologies, Böblingen, Germany). Only samples with RIN (RNA integrity number) of 5.0 or higher were subjected to gene expression array analysis.

### Illumina microarray experiments and data analysis

Global gene expression screenings were performed using Biobank samples only (steatohepatitis n=8 (6/2 cirrhotic/non-cirrhotic), steatosis n=14 and controls n=10). For gene expression analysis, whole genome expression microarray Sentrix^{®} Human-6 v3 expression bead chip (Illumina^{®}, San Diego, CA, USA) encompassing 49577 features was used. The experiments were performed at the Genomics and Proteomics Core Facility of DKFZ Heidelberg using 300 ng/µl RNA and protocols recommended by the supplier. The raw data was log2-transformed and quantile-normalized. Principal Components Analysis (PCA) was performed to investigate the internal data structure in a way which best explained the variance in the data and to identify potential outliers. The R-package "limma" was used to identify differentially expressed genes (Smyth, Stat Appl Genet Mol Biol 3 (2004): Article 3). With "limma", a linear model is fitted for each gene and a t-test is used to identify differential expressed genes. P-values were adjusted for multiple testing using the Benjamini-Hochberg (BH) procedure. In addition, a fold change of at least 30% was required to designate a gene as differentially expressed. Resulting data was imported into Ingenuity Pathway analysis (IPA) software (Ingenuity Systems, Redwood City, CA, USA) to identify over- or underrepresented pathways as well as potential biomarkers.

### Quantitative real-time PCR

Quantitative gene expression analysis was performed with 87 human liver tissue samples (Biobank samples: 10 steatohepatitis, 8/2 cirrhotic/non-cirrhotic; 30 steatosis and 18 controls; Biopsy samples: 11 steatohepatitis, 5/6 cirrhotic/non-cirrhotic; 13 steatosis; 5 disease controls from patients with chronic hepatitis C) using real-time PCR (LightCycler^{®} 480 Real-Time PCR System, Roche Applied Science, Mannheim, Germany). Gene specific primer and probe TaqMan^{®} gene expression assays (Applied Biosystems, Weiterstadt, Germany) were applied and performed relative quantification of all genes. HPRT1 was used as a house-keeping gene to normalize the data. The Cp value was extracted from the Lightcycler^{®} 480 software 1.5.0 and the expression level was calculated with the 2-ΔCp method (Pfaffl, Nucleic Acids Res 29 (2001): e45; Livak et al., Method. Methods 25 (2001): 402-408).

### Immunohistochemistry

For immunohistochemical analysis 3 µm thick paraffin sections of liver tissue of one case each of chronic hepatitis C, NAFLD associated steatosis (NAFL) and steatohepatitis can be dewaxed and rehydrated following standard procedures. For antigen retrieval the sections can be microwaved in Target Retrieval Solution, pH 9.0 (Dako REAL TM S2367; Dako, Glostrup, Denmark), for 40 min at 160 W followed by cooling down for 20 min at RT. The sections can then be washed in water and PBS. Blocking can be carried out with Dako REAL TM Blocking Solution for 10 min prior to incubation with antibodies against the aldose reductase AKR1B10 diluted 1:500 in Dako REAL TM Antibody Diluent for 60 min at RT. Binding of the antibodies can be detected with the Dako REAL TM EnVision TM Detection System Peroxidase/DAB+. KRT23 protein expression detected in the cytoplasm of hepatocytes can be assessed semiquantitatively. The intensity of immunostaining can be classified as mild to moderate or marked, and the amount of positive hepatocytes can be estimated by application of numerical scores which can be defined as: Score 0 - AKR1B10 immunostaining in hepatocytes not detected; Score 1 - AKR1B10 positive hepatocytes comprise less than 10% of liver parenchyma; Score 2 - AKR1B10 positive hepatocytes comprise between 10-30% of liver parenchyma; Score 3 - AKR1B10 positive hepatocytes comprise more than 30% of liver parenchyma.

### Results

### Gene expression pattern clearly separates steatohepatitis from steatosis and controls

To screen for changes in hepatic gene expression distinguishing steatohepatitis from steatosis, liver samples from patients with both alcoholic and nonalcoholic fatty liver disease obtained by surgery (Biobank) were subjected to Illumina gene expression bead chip-based analysis (table 1). A total of 32 Biobank samples (table 1 and experimental procedure) were investigated. Gene expression data were subsequently explored by two data analysis procedures, consisting of (i) identification of differentially expressed genes using limma and (ii) selection of the 1000 genes with the highest variance in the entire data set.

In the pair-wise comparisons, 4963 and 2543 genes were identified as significantly (FDR <0.05) differentially expressed between steatohepatitis and controls, and steatohepatitis and steatosis, respectively. The differences between the gene expression profiles of the normal and steatosis samples are small. The variance within the groups is high and due to that, the power of the statistical test is not sufficient to identify significantly differentially expressed genes between the two groups. The number of differentially expressed genes indicate that steatohepatitis, when compared to normal liver tissue, is characterized by more profound molecular changes than steatosis. The two comparisons, steatohepatitis vs. steatosis and steatohepatitis vs. controls, shared 1931 differentially expressed genes.

Hierarchical clustering was used to visualize these common genes in a heat map (fig. 1A). With two exceptions steatosis and control samples clustered together whereas steatohepatitis samples clustered in a separate branch. The common genes were divided into two branches, down-regulated genes in steatohepatitis compared to steatosis and controls (fig. 1A, cluster 1) or upregulated in steatohepatitis compared to steatosis and controls (fig. 1A, cluster 2).

The 1000 genes with the highest variance were used for an unsupervised clustering and a visualization of the results in a heat map (fig. 1B). Again, with one exception, steatohepatitis samples clustered in a branch separated from steatosis samples and controls. The highly variant genes fell into two classes, characterized by up- and down-regulation in steatohepatitis compared to the remaining samples. Taken together, both types of analyses yielded differential gene expression signatures, which clearly separated steatohepatitis from steatosis and control samples.

Gene ontology (Alexa et al., Bioinformatics 22 (2006): 1600-1607) analysis was performed for cluster 1 and cluster 2 (table 2 A and B, respectively) to identify disease-relevant biological processes from differentially expressed genes. Notably, cluster 1 (upregulated in controls and steatosis) was characterized by metabolism only (oxidation reduction, metabolic processes, and biosynthesis). In contrast, several GO classes in cluster 2 (upregulated in steatohepatitis) belonged to molecular processes, which are characteristic for malignant diseases and tumour progression (cell adhesion, extracellular matrix, cell motion, integrin signalling). This finding suggests that steatohepatitis is accompanied by entirely different gene expression programs when compared to steatosis and controls. Notably, the programs upregulated in steatohepatitis are characteristic for malignant diseases.

### qRT-PCR validation of microarray data

A total of 58 biobank samples were used for PCR-based validation of the array data. To optimally select candidate genes for validation, three strategies were employed. First, 265 common genes between the 1931 differentially expressed genes and the 1000 genes with the highest variation were identified. This group of common genes was analyzed by the web-based software IPA^{®} (Ingenuity^{®} systems). IPA Biomarker^{®} was applied to identify relevant biomarker candidates that could be useful to distinguish between steatohepatitis and steatosis. A total of 126 genes were detected after filtering. From this group, 13 genes (table 3) were selected for further validation, due to their significant fold change in the microarray analysis. Second, GO terms from the two defined clusters were systematically analyzed for potential targets and three genes were chosen (table 3). Third, 17 differentially expressed genes (table 3) were identified by a significant p-value and fold change from the microarray data. Furthermore, five genes (table 3) were used as positive controls and additionally, eight genes (table 3) were chosen due to known function on protein level in steatohepatitis. Within the selection process of applicable candidates, main focus was made on genes linked to metabolism, oxidative stress, inflammation, and their relevance in fatty liver disease. In summary, 46 genes were selected for further validation by qRT-PCR. *HPRT1* was used as a housekeeping gene. Figure 2 summarizes the fold changes of the expression of the selected genes. The rates of verification of differential gene expression were high: The changes - as determined by qRT-PCR - of differential expression for 39 genes in Biobank steatohepatitis samples were significant. Only three genes were not significantly deregulated. In biopsies, 30 genes were significant, and twelve genes were not significantly deregulated. The reasons for the lower verification rate in biopsies may be that most of the analyzed Biobank samples had already been used for the whole genome microarray experiment (no independent sample cohort). Four genes (DCDC2, EEF1A2, GSTM1 and STMN2) could not be reliably measured in any of the samples.

The genes ACSL4, ATF3, CAT, GSTM5 and NROB2 were previously described in fatty liver disease and served as positive controls for the quantitative validation of candidate genes in the present study. The significant up-regulation of these positive control genes was validated in either one or both of the analyzed cohorts (table 3).

The major finding supports the notion of a striking alteration of molecular programs in steatohepatitis when compared to steatosis and control samples. The present data showed a highly significant overexpression of KRT23 in steatohepatitis (see figure 3 concerning the validation of KRT23 mRNA expression by qRTPCR in biopsy (A) and biobank (B) samples; HPRT1 was chosen to normalize the gene expression in the analyzed samples; mean normalized expression levels are given in log2).

### Discussion

The present study unravels gene expression signatures profoundly distinguishing steatohepatitis from steatosis and normal liver. Notably, normal tissue and steatosis clustered more closely together when compared to the steatohepatitis samples. The gene expression data suggests that steatohepatitis exhibits molecular profiles which are characteristic for processes relevant in malignant tumours and may therefore reflect a premalignant state of liver disease already at the precirrhotic stage. Indeed, increasing evidence supports the fact that steatohepatitis can progress to HCC already in precirrhotic stage. Obesity and diabetes are not only established risk factors for developing steatohepatitis and cirrhosis, but have also been implicated in the formation of HCC. Other major risk factors for HCC in steatohepatitis are advanced age and tissue fibrosis.

The mechanisms underlying the progression from steatohepatitis to HCC are still unclear, and targets for the treatment of steatohepatitis and HCC are missing. However, signalling pathways involved in inflammation and insulin resistance promoting steatohepatitis, may contribute to its carcinogenic potential.

The gene expression of the 46 selected genes was validated by qRT-PCR for both of the tested sample cohorts. Although the analyzed samples were collected by different approaches (comprising both alcoholic and non-alcoholic as well as cirrhotic and non-cirrhotic steatohepatitis samples, table 1) and for the biopsy samples chronic hepatitis C samples were used as controls (since percutaneous liver biopsy is not performed in individuals with normal liver), many genes were significantly deregulated between the three tested groups of liver samples in both independent sample cohorts. This underlines common pathogenetic mechanisms in both alcoholic and non-alcoholic steatohepatitis. Among the differentially expressed genes, AKR1B10 and KRT23 were the most prominent ones. AKR1B10 belongs to the aldose reductases and was first described in human HCC. It is a monomeric enzyme reducing aldehydes and ketones to the corresponding alcohols. The protein is expressed in cervical cancer and non-small cell lung cancer, where it is considered as a potential diagnostic biomarker. Several findings also support the hypothesis that AKR1B10 may be a useful marker for differentiation and proliferation of liver, colon, lung and breast cancer. In addition, AKR1B10 protein plays a role in the detoxification of toxic aldehydes. Free radicals generated by reactive oxygen species oxidize fatty acids of the lipid membrane resulting in the production of reactive aldehydes, which are rapidly reduced by AKR1B10 thereby protecting cells from toxification. Lipotoxicity of fatty acid and lipid intermediary metabolites may be a key event in the progression of fatty liver disease by inducing hepatocellular death. In the present study, the significant overexpression of AKR1B10 in steatohepatitis is shown which could be caused by the increased need to inactivate toxic components in hepatocytes. This suggests that AKR1B10 may be a molecular marker accompanying the progression of steatohepatitis to HCC.

Genes involved in lipid partitioning by keeping potentially lipotoxic fatty acids stored in neutral triglycerides (TG) may counteract/protect from lipotoxicity as potentially important factor in progression of NAFLD to steatohepatitis. Notably, some of the genes which were found to be deregulated included enzymes involved in FA homeostasis such as DGAT2 (responsible for FA esterification to TGs) and PNPLA3 (adiponutrin), recently identified as key determinant for pathogenesis and progression of alcoholic and non-alcoholic fatty liver disease.

Furthermore, according to the present invention, a new and highly reliable marker is provided. The present invention has revealed the highly significant overexpression of KRT23 in steatohepatitis compared to steatosis and normal liver. KRT23 has been detected in different cancer types. In microsatellite stable colon cancer, KRT23 expression is highly increased, and this may have a protective function counteracting the proliferation and survival of cells. Another study identified KRT23 protein as a HCC-associated antigen in patient sera. KRT23 has not yet been described to be expressed in liver or liver disease. The role of KRT23 under physiological conditions and in steatohepatitis is unknown. However, mutations of other members of the keratin multigene family, namely keratins 8 and 18 were found to be associated with chronic human liver disease. Furthermore, disturbances of the expression levels of keratins 8 and 18 led to formation of Mallory-Denk bodies, an important morphological characteristic of steatohepatitis. Nevertheless, the functional role of KRT23 in liver or liver diseases needs to be resolved.

In summary, the present data show that that KRT23 represents a highly reliable pre-malignant marker in the progression of steatohepatitis to HCC. These marker genes can be useful to test patients for a potential development of HCC and for therapeutic decisions in clinical practice.

**Table 1: Clinical, biochemical and histopathological patient characteristics. (p-value: a. steatohepatitis vs. steatosis, b. steatohepatitis vs. control, c. steatosis vs. control)**

| **Microarray samples** | | | | |
|---|---|---|---|---|
| | Steatohepatitis (n=8) | Steatosis (n=14) | Controls (n=10) | p-value |
| **Sex (m:f)** | 6:2 | 8:6 | 5:5 | |
| **Age (y)** | 55 (46-72) | 61.5 (37-78) | 51 (25-73) | 0.37 |
| **BMI (kg/m2)** | 25.5 (19.8-39.2) | 31 (21.4-30.8) | 22.8 (18.3-30.1) | 0.17 |
| **ALT (IU/1)** | 34 (16-56) | 25 (12-359) | 20 (5-156) | 0.57 |
| **AST (IU/1)** | 59 (38-92) | 27 (9-398) | 22 (10-240) | 0.02 a,b |
| **GGT (IU/1)** | 60 (24-804) | 46 (14-136) | 36 (15-146) | 0.26 |
| **AP (IU/1)** | 88 (60-261) | 88 (42-146) | 77 (45-157) | 0.55 |
| **Bilirubin (mg/dl)** | 3.2 (0.8-7.8) | 0.8 (0.3-2.5) | 1 (0.2-2) | 0.01 a,b |
| **Cholesterol (mg/dl)** | 112 (57-177) | 226 (117-270) | 174 (44-240) | 0.00 a |
| **Triglycerides (mg/dl)** | 97 (57-203) | 159 (117-270) | 97 (32-172) | 0.04 a,c |
| **Diabetes II (Y/N)** | 2/6 | 3/11 | 0/10 | |
| **Hyperlipidaemia (Y/N)** | 2/6 | 5/9 | 0/10 | |
| **Hypertension (Y/N)** | 6/2 | 8/6 | 0/10 | |
| **Histological steatosis grade** | 3:1:4:0 | 0:10:3:1 | 10:0:0:0 | |
| **(0:1:2:3)** | | | | |
| **Lobular inflammation** | 1:3:3:1 | 1:7:3:1 | 6:3.1:0 | |
| **(0:A1:A2:A3)** | | | | |
| **Ballooning (0:1:2)** | 0:4:4 | 14:0:0 | 10:0:0 | |
| **NAS (points)** | 4 (1-7) | 2 (1-6) | n/a | |
| **Matteoni (points)** | 3 (0-4) | 2 (1-2) | n/a | |
| **Fibrosis** | 0:0:0:2:6 | 9:4:1:0:0 | 9:1:0:0:0 | |
| **(0:F1:F2:F3:F4)** | | | | |
| **Apoptosis (0:1:2)** | 4:4:0 | 7:5:2 | 6:4:0 | |
| **Alcoholic/non-alcoholic** | 5:3 | 0:14 | n/a | |

| **qRTPCR biobank samples** | | | | |
|---|---|---|---|---|
| | Steatohepatitis (n=10) | Steatosis (n= 30) | Controls (n=18) | p-value |
| **Sex (m:f)** | 7:03 | 14:16 | 9:09 | |
| **Age (y)** | 54 (44-72) | 64 (37-78) | 52.5 (22-73) | 0.02 c |
| **BMI (kg/m2)** | 26.1 (19.8-39.2) | 25.7 (21-33.5) | 23.2 (18.3-30.1) | 0.03 c |
| **ALT (IU/1)** | 39 (16-204) | 25 (11-359) | 21.5 (5-311) | 0.33 |
| **AST (IU/1)** | 65 (38-441) | 28 (9-398) | 23.5 (7-240) | 0.00 a,b |
| **GGT (IU/1)** | 98 (24-804) | 40 (14-286) | 35 (9-223) | 0.03 a,b |
| **AP (IU/1)** | 132 (60-342) | 87 (42-161) | 77 (45-157) | 0.14 |
| **Bilirubin (mg/dl)** | 3.43 (0.8-8.5) | 0.72 (0.3-3.3) | 0.87 (0.2-2.4) | 0.00 a,b |
| **Cholesterol (mg/dl)** | 114 (57-180) | 219 (87-301) | 160 (44-240) | 0.00 a,c |
| **Triglycerides (mg/dl)** | 103 (57-231) | 149 (54-712) | 95.5 (10-184) | 0.02 c |
| **Diabetes II (Y/N)** | 2/8 | 8/22 | 1/17 | |
| **Hyperlipidaemia (Y/N)** | 3/7 | 7/23 | 1/17 | |
| **Hypertension (Y/N)** | 3/7 | 18/12 | 1/17 | |
| **Histological Steatosis grade** | 3:1:5:1 | 0:19:8:3 | 17:0:0:0 | |
| **(0:1:2:3)** | | | | |
| **Lobular inflammation** | 2:3:3:2 | 2:18:9:1 | 11:5:1:0 | |
| **(0:A1:A2:A3)** | | | | |
| **Ballooning (0 : 1 : 2)** | 0 : 5 : 5 | 30 : 0 : 0 | 17:0:0 | |
| **NAS (points)** | 4 (1-8) | 2 (1-6) | n/a | |
| **Matteoni (points)** | 4 (0-4) | 2 (1-3) | n/a | |
| **Fibrosis** | 0 : 0 : 0 : 2 : 8 | 23 : 6 : 1 : 0 : 0 | 11 : 5 : 1 : 0 : 0 **(0 : F1 : F2 : F3 : F4)** | |
| **Apoptosis (0 : 1 : 2)** | 4 : 6 : 0 | 9 : 15 : 6 | 12:5:0 | |
| **Alcoholic/non-alcoholic** | 7:3 | 0 : 30 | n/a | |

| **qRTPCR biopsy samples** | | | | |
|---|---|---|---|---|
| | Steatohepatitis (n=11) | Steatosis (n=13) | Controls (n=5) | p-value |
| **Demographics. s.o.** | | | | |
| **sex (m:f)** | 6:05 | 9:04 | 4:01 | |
| **age (y)** | 54 (34-69) | 45 (25-62) | 43 (28-50) | 0.07 |
| **BMI (kg/m2)** | 28.8 (25.2-31.6) | 28.3 (22.8-35.5) | 26.7 (24.3-27.7) | 0.19 |
| **ALT (IU/1)** | 83 (43-154) | 75 (21-253) | 66 (39-130) | 0.59 |
| **AST (IU/1)** | 68 (35-520) | 42 (19-137) | 41 (33-65) | 0.07 |
| **GGT (IU/1)** | 426 (98-2195) | 171 (17-536) | 40 (27-69) | 0.00 a,c |
| **AP (IU/1)** | 118 (76-267) | 87 (37-224) | 55 (38-69) | 0.01 b |
| **Bilirubin (mg/dl)** | 1 (0.4-1.7) | 0.68 (0.3-3.6) | 0.46 (0.4-1.5) | 0.27 |
| **Cholesterol (mg/dl)** | 258 (131-326) | 206 (146-259) | 142 (125-177) | 0.04 b,c |
| **Triglycerides (mg/dl)** | 185 (127-488) | 161 (51-324) | 43 (20-56) | 0.01 b,c |
| **Diabetes II (Y/N)** | 4/7 | 3/8 | 0/5 | |
| **Hyperlipidaemia (Y/N)** | 3/8 | 3/8 | 0/5 | |
| **Hypertension (Y/N)** | 5/6 | 4/7 | 0/5 | |
| **Steatosis grade (0:1:2:3)** | 0:3:3:4 | 0:5:3:3 | 4:1:0:0 | |
| **Lobular inflammation** | 0 : 2 : 5 : 3 | 1 : 5 : 5 : 0 | n/a | |
| **(0 : A1 : A2 : A3)** | | | | |
| **Ballooning** | (0:1:2) 0 : 9.1 | 11:0:0 | 5:0:0 | |
| **NAS (points)** | 5 (4-8) | 3 (1-5) | n/a | |
| **Matteoni (points)** | 4 (2-4) | 2 (1-2) | n/a | |
| **Fibrosis** | 0:5:1:0:4 | 7:2:1:0:1 | n/a **(0 : F1 : F2 : F3 : F4)** | |
| **Apoptosis (0:1:2)** | 4:6:0 | 8 : 3 : 0 | n/a | |
| **Alcoholic/non-alcoholic** | 3:8 | 0:13 | n/a | |

**Table 2: Gene Ontology (GO) analysis. A. Over-represented GO terms for down regulated genes in the common gene list in steatohepatitis samples (Fig. 1A, cluster 1). B. Over-represented GO terms for up regulated genes in common gene list in steatohepatitis samples (Fig. 1A, cluster 2).**

| A. | | | |
|---|---|---|---|
| | GO.ID | Term | pvalue |
| 1 | GO : 0006805 | xenobiotic metabolic process | 3.0e-07 |
| 2 | GO : 0055114 | oxidation-reduction process | 3.7e-07 |
| 3 | GO : 0032787 | monocarboxylic acid metabolic process | 7.1e-07 |
| 4 | GO : 0006569 | tryptophan catabolic process | 1.9e-05 |
| 5 | GO : 0042559 | pteridine-containing compound biosynth. process | 7.5e-05 |
| 6 | GO : 0009437 | carnitine metabolic process | 8.9e-05 |
| 7 | GO : 0046874 | quinolinate metabolic process | 0.0002 |
| 8 | GO : 0070646 | protein modification by small protein removal | 0.0005 |
| 9 | GO : 0016098 | monoterpenoid metabolic process | 0.0008 |
| 10 | GO : 0006542 | glutamine biosynthetic process | 0.0012 |
| | | | |

| B. | | | |
|---|---|---|---|
| | GO.ID | Term | pvalue |
| 1 | GO : 0007155 | cell adhesion | 1.7e-15 |
| 2 | GO : 0006415 | translational termination | 1.7e-10 |
| 3 | GO : 0006414 | translational elongation | 1.8e-10 |
| 4 | GO : 0006935 | chemotaxis | 2.7e-10 |
| 5 | GO : 0007409 | axonogenesis | 4.4e-10 |
| 6 | GO : 0030198 | extracellular matrix organization | 8.4e-09 |
| 7 | GO : 0019083 | viral transcription | 2.5e-08 |
| 8 | GO : 0016477 | cell migration | 1.0e-07 |
| 9 | GO : 0031018 | endocrine pancreas development | 1.0e-07 |
| 10 | GO : 0042060 | wound healing | 1.1e-06 |

**Table 3. Candidate genes for qRT-PCR validation.FCs (fold changes) of KRT23 gene were calculated from gene expression array data. Gene expression from selected target genes was compared between steatohepatitis (SH) and controls (ctrl) as well as between SH and steatosis.**

| | |
|---|---|
| **Chip/FC (SH/ctrl)** | 1.261 |
| **Chip/FC (SH/steatosis)** | 1.253 |
| **FC q-RTPCR (SH/ctrl)** | 460.482 |
| **p-value (SH/ctrl)** | 2.29E-10 |
| **FC q-RT-PCR (SH/steatosis)** | 155.130 |
| **p-value (SH/steatosis)** | 9.57E-09 |
| **FC q-RT-PCR (steatosis/ctrl)** | 2.968 |
| **p-value (steatosis/ctrl)** | 0.011 |
| **FC q-RT-PCR (SH/ctrl)** | 39.302 |
| **p-value (SH/ctrl)** | 0.001 |
| **FC q-RT-PCR (SH/steatosis)** | 14.809 |
| **p-value (SH/steatosis)** | 0.006 |
| **FC q-RT-PCR (steatosis/ctrl)** | 2.654 |
| **p-value (steatosis/ctrl)** | 0.049 |

## Claims

1. Use of keratin 23 (KRT23) as a marker for distinguishing between steatosis and steatohepatitis.

2. A method for diagnosing steatohepatitis in a sample of a human body fluid or a human tissue sample comprising the determination of the amount of KRT23 or KRT23 m-RNA in this sample and diagnosing steatohepatitis, if the amount of KRT23 or KRT23 m-RNA is increased compared to a sample of this human body fluid or tissue sample from a person with no steatohepatitis.

3. A method according to claim 2, wherein the body fluid is blood or a blood derived sample, preferably a serum or a plasma sample.

4. A method according to claim 2, wherein the tissue sample is a liver tissue sample or a liver biopsy sample.

5. A method according to any one of claims 2 to 4, wherein the amount of KRT23 in this sample is detected by a KRT23 antibody.

6. A method according to any one of claims 2 to 4, wherein the amount of KRT23 m-RNA in this sample is detected by a KRT23 m-RNA binding nucleic acid.

7. KRT23 for use in a method for diagnosing steatohepatitis.

8. Use of a kit for detecting the expression level of KRT23 in a sample, comprising detection agents for KRT23 mRNA for diagnosing a tissue sample or a sample of a body fluid for steatohepatitis.

9. Use of a kit for detecting the amount of KRT23 in a sample, comprising detection agents for KRT23 for diagnosing a tissue sample or a sample of a body fluid for steatohepatitis.

10. A diagnostic kit for diagnosing steatohepatitis in a tissue sample or a sample of a body fluid, comprising
- a KRT23 m-RNA binding agent, preferably a KRT23 m-RNA binding nucleic acid and/or
- a KRT23 binding agent, preferably a KRT23 antibody.
